(19) Europäisches Patentamt
European Patent Office
Office européen des brevets

(11) Veröffentlichungsnummer: **0 217 101 A1**

(12) # EUROPÄISCHE PATENTANMELDUNG

(21) Anmeldenummer: 86111563.2

(22) Anmeldetag: 21.08.86

(51) Int. Cl.[4]: C 07 C 79/46
C 07 C 76/00, C 07 C 121/34
C 07 C 120/00, C 07 C 103/34
C 07 C 102/00, C 07 C 149/20
C 07 F 9/40, C 07 D 317/32
C 07 D 277/24, C 07 C 147/14

(30) Priorität: 30.08.85 DE 3531006

(43) Veröffentlichungstag der Anmeldung: 08.04.87 Patentblatt 87/15

(84) Benannte Vertragsstaaten: AT BE CH DE FR GB IT LI NL SE

(71) Anmelder: HOECHST AKTIENGESELLSCHAFT
Postfach 80 03 20
D-6230 Frankfurt am Main 80(DE)

(72) Erfinder: Schlegel, Günter, Dr.
Am Flachsland 54
D-6233 Kelkheim (Taunus)(DE)

(72) Erfinder: Mildenberger, Hilmar, Dr.
Fasanenstrasse 24
D-6233 Kelkheim (Taunus)(DE)

(72) Erfinder: Bauer, Klaus, Dr.
Kolpingstrasse 7
D-6054 Rodgau(DE)

(72) Erfinder: Bieringer, Hermann, Dr.
Eichenweg 26
D-6239 Eppstein/Taunus(DE)

(54) **Neue substituierte Diphenylether, Verfahren zu ihrer Herstellung und ihre Verwendung als Herbizide.**

(57) Die Verbindungen der Formel (I)

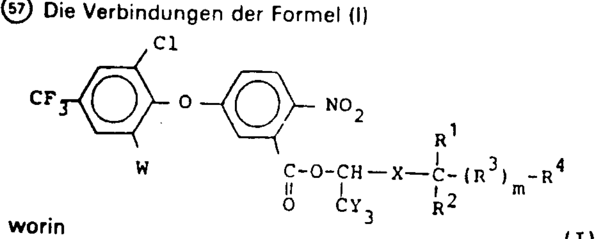

worin (I),

$R^1$, $R^2$ = H, (subst.) Alkyl, (subst.) Phenyl, (subst.) Thienyl, (subst.) Furanyl, einen Rest der Formeln

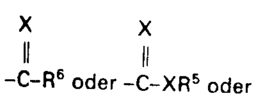

$R^1$, $R^2$ zusammen mit dem benachbarten C-Atom einen (subst.) (-O- oder -CO-haltigen) Kohlenwasserstoffring,

$R^3$ = einen Rest der Formeln $-(C(R^5)_2)_n-$, $-(CH_2)_n-X-(CH_2)_p-$ oder $-X-$,

$R^4$ = H, (subst.) Alkyl, einen (subst.) Thiazolyl- oder Dioxolan-Rest, einen Rest der Formel

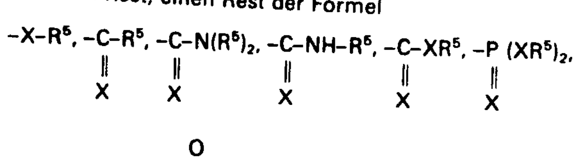

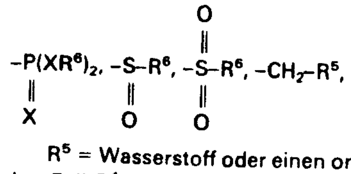

$R^5$ = Wasserstoff oder einen organischen Rest, sowie für den Fall $R^4$ = $-C(X)XR^5$ oder $-P(X)(XR^5)_2$ ein in der Landwirtschaft einsetzbares Kation,

$R^6$ = einen organischen Rest, W=Wasserstoff, Chlor oder Fluor, X=O, S oder $NR^5$, Y=Halogen oder Wasserstoff, m=O oder 1, n=1, 2 oder 3 und p=0, 1, 2 oder 3 bedeuten, besitzen vorteilhafte herbizide Wirkungen gegen mono- und dikotyle Schadpflanzen.

EP 0 217 101 A1

Croydon Printing Company Ltd

Neue substituierte Diphenylether, Verfahren zu ihrer Herstellung und ihre Verwendung als Herbizide

Es ist bereits bekannt, daß substituierte Diphenylether herbizide Eigenschaften aufweisen, siehe z.B. EP-A-34 883, US-PS 4,429,146.
Diese weisen jedoch Nachteile bei der Anwendung auf, wie beispielsweise mangelnde Selektivität oder auch zu geringe Wirkungsbreiten.

Es wurde nun gefunden, daß Diphenylether, die eine mit einem Halbacetal, Halbthioacetal oder Halbaminal veresterte Carboxylgruppe tragen, vorteilhafte herbizide Wirkungen besitzen.

Gegenstand der vorliegenden Erfindung sind daher Verbindungen der Formel (I)

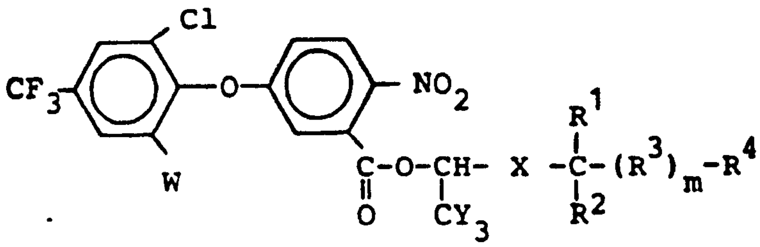

(I)

worin

$R^1$, $R^2$ unabhängig voneinander Wasserstoff, $(C_1-C_4)$ Alkyl, das durch Halogen, Nitro, Cyano, $(C_1-C_4)$ Alkoxy, $(C_1-C_4)$ Alkylthio, einen Rest der Formeln $-COR^6$, $-O-CO-R^6$ oder $-N(R^5)_2$ ein- oder mehrfach substituiert sein kann, Phenyl, Thienyl, Furanyl, die alle drei durch $(C_1-C_4)$ Alkyl, Halogen, Nitro, Cyano, einen Rest der Formeln $-COOR^5$, $-OR^5$, $-S-R^5$, $-N(R^5)_2$ oder $-PO(XR^5)_2$ ein- oder mehrfach, insbesondere ein- bis dreifach, substituiert sein können,

einen Rest der Formeln 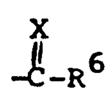 oder 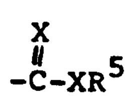 oder

$R^1$, $R^2$ zusammen mit dem benachbarten C-Atom einen vier- bis

siebengliedrigen gesättigten Kohlenwasserstoffring, bei dem eine oder zwei Methylengruppen durch $>C=O$ und/oder -O- ersetzt sein kann und welcher durch $(C_1-C_4)$ Alkyl ein- oder mehrfach substituiert sein kann,

$R^3$ einen Rest der Formeln $-(C(R^5)_2)_n-$, $-(CH_2)_n-X-(CH_2)_p-$ oder -X-

$R^4$ Wasserstoff, $(C_1-C_4)$ Alkyl, das durch Halogen, Cyano, Nitro, $(C_1-C_4)$ Alkylsulfinyl oder $(C_1-C_4)$ Alkylsulfonyl ein- oder mehrfach, insbesondere ein- bis dreifach (außer im Falle Halogen), substituiert sein kann, einen Thiazolyl- oder Dioxolan-Rest, die beide durch $(C_1-C_4)$ Alkyl und/oder $(C_1-C_4$ Alkoxy)-carbonyl substituiert sein können, einen Rest der Formel $-X-R^5$,

$-\underset{\underset{X}{\|}}{C}-R^5$, $-\underset{\underset{X}{\|}}{C}-N(R^5)_2$, $-\underset{\underset{X}{\|}}{C}-NH-R^5$, $-\underset{\underset{X}{\|}}{C}-XR^5$,

$-\underset{\underset{X}{\|}}{P}(XR^5)_2$, $-\underset{\underset{X}{\|}}{P}(R^6)_2$, $-\underset{\underset{O}{\|}}{S}-R^6$, $-\overset{\overset{O}{\|}}{\underset{\underset{O}{\|}}{S}}-R^6$, $-CH_2-R^5$,

$R^5$ = Wasserstoff, $(C_1-C_4)$ Alkyl, $(C_2-C_6)$ Alkenyl, $(C_2-C_6)$ Alkinyl, wobei die drei letztgenannten Reste durch Halogen, Nitro, Cyano, $(C_1-C_4)$ Alkoxy, $(C_1-C_4)$ Alkylthio, Di $(C_1-C_4$-alkyl)-amino, $(C_1-C_4$-Alkoxy)-carbonyl, Tri $(C_1-C_4$-alkyl)silyl, Phenyl, Phenyl, das durch $(C_1-C_4)$ Alkyl, Halogen, Nitro, Cyano, Carboxy, $(C_1-C_4$-Alkoxy)carbonyl, $(C_1-C_4)$ Alkoxy oder Di-$(C_1-C_4$-alkyl) amino substituiert ist, Furanyl, Thienyl oder Furanyl, Thienyl, die beide durch $(C_1-C_4)$ Alkyl, Halogen oder $(C_1-C_4)$ Alkoxy substituiert sind, substituiert sein können,

Phenyl oder ein heterocyclischer 5 oder 6-Ring mit ein bis drei Heteroatomen aus der Gruppe Sauerstoff, Schwefel, Stickstoff als Ringglieder, wobei Phenyl

oder der heterocyclische Ring durch $(C_1-C_4)$ Alkyl, Halogen, Nitro, Cyano, $(C_1-C_4)$ Alkoxy, $(C_1-C_4)$ Alkylthio, $(C_1-C_4$-Alkoxy)carbonyl, Carboxy, Amino, $(C_1-C_4)$ Alkylamino, Di$(C_1-C_4$-alkyl)amino oder $-PO(O-C_1-C_4\text{-alkyl})_2$ substituiert sein kann, sowie für den Fall $R^4 = -C(X)XR^5$ oder $P(X)(XR^5)_2$ ein in der Landwirtschaft einsetzbares Kation,

$R^6$ die für $R^5$ genannten Reste außer Wasserstoff

W Wasserstoff, Chlor oder Fluor,

X O, S oder $NR^5$,

Y Halogen oder Wasserstoff,

m 0 oder 1

n 1,2 oder 3 und

p 0, 1, 2 oder 3 bedeuten.

Halogen bedeutet bevorzugt Fluor, Chlor, Brom.

Die Verbindungen der Formel I können als Stereoisomerengemische oder in Form reiner Stereoisomeren vorliegen, da sie je nach Substitution ein oder mehrere chirale Zentren besitzen. Diese Stereoisomeren und deren Gemische werden alle von vorliegender Erfindung umfaßt.

Als Heterocyclen für $R^5$, $R^6$ kommen beispielsweise in Frage: Thiophen, Furan, Pyridin, Pyrrol, Thiazol, Triazol, Pyridin.

Als Kationen für $R^5$ kommen beispielsweise in Frage: ein Na-, K-, Ca-, Mg-, Ammonium-Kation oder ein durch einen üblichen organischen Rest einfach oder mehrfach substituiertes Ammonium-Kation.

$R^1$, $R^2$ bedeuten insbesondere Wasserstoff oder $(C_1-C_4)$Alkyl, das, wie oben angegeben, substituiert sein kann, wobei die Substituenten außer im Falle Halogen insbesondere ein- bis dreifach auftreten können; $R^5$ besitzt insbesondere die Bedeutung von Wasserstoff oder $(C_1-C_4)$Alkyl, das wie oben angegeben substituiert sein kann, wobei für $R^3 = -(C(R^5)_2)_n-$ als Substituenten insbesondere Halogen, Cyano oder $(C_1-C_4)$-Alkoxy von Bedeutung sind.

Besonders bevorzugt sind solche Verbindungen der Formel I, worin $R^1$ = H, $R^2$ = H, $R^4$ einen Rest der Formeln $-C(X)XR^5$ oder $-P(O)(XR^5)_2$, $R^5$, $R^6$ = ($C_1$-$C_4$) Alkyl oder Benzyl, X = O oder S, W = H oder Cl, Y = Cl oder F und m = 0 bedeuten.

Gegenstand der vorliegenden Erfindung ist auch ein Verfahren zur Herstellung der Verbindungen der Formel I, dadurch gekennzeichnet, daß man eine Verbindung der Formel (II)

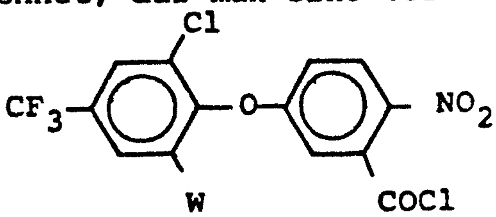

(II)

mit einer Verbindung der Formel (III)

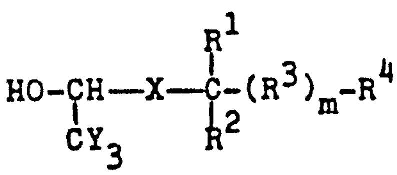

(III)

umsetzt und den entstehenden Chlorwasserstoff aus dem Reaktionsgemisch entfernt.

Der Chlorwasserstoff kann beispielsweise mit einer üblichen Base wie NaOH abgefangen oder aus der Reaktionslösung mittels Inertgas ausgeblasen werden.

Die Umsetzung der Verbindungen (II) und (III) erfolgt auf üblichem Wege vorzugsweise in inerten aprotischen Lösungsmitteln, wie z.B. Acetonitril, Dichlormethan, Toluol, Tetrahydrofuran oder Dioxan bei Temperaturen zwischen -20°C und der Siedetemperatur des verwendeten Lösungsmittels, vorzugsweise bei -5°C bis +25°C.

Die Diphenylether-Säurechloride der Formel (II) lassen sich nach bekannten Methoden darstellen (siehe z.B. Houben-Weyl, Bd. VI/3, S. 85ff (1965), EP-A-80312, US-PS 4 031 131).

Die Alkohole der Formel III lassen sich aus Alkoholen, Thiolen oder Aminen durch Umsetzung mit Aldehyden unter prinzipiell bekannten Reaktionsbedingungen herstellen (vgl. J. prakt.Chem. 316, 304 (1974), Indian J.Chem. 13, 854 (1975),

Bull.Soc.Chim.Fr. 47, 354 (1930)).

Die erfindungsgemäßen Verbindungen der Formel I weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es gleichgültig, ob die Substanzen im Vorsaat-, Vorauflauf oder Nachauflaufverfahren ausgebracht werden.

Werden die erfindungsgemäßen Verbindungen vor dem Keimen auf die Erdoberfläche appliziert, so wird entweder das Auflaufen der Unkrautkeimlinge vollständig verhindert, oder die Unkräuter wachsen bis zum Keimblattstadium heran, stellen jedoch dann ihr Wachstum ein und sterben schließlich nach Ablauf von drei bis fünf Wochen vollkommen ab.

Bei Applikation der Wirkstoffe auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit mehr oder weniger schnell ganz ab, so daß auf diese Weise eine für die Kulturpflanzen schädliche Unkrautkonkurrenz sehr früh und nachhaltig durch den Einsatz der neuen erfindungsgemäßen Mittel beseitigt werden kann.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z.B. Weizen, Gerste, Roggen, Reis, Mais, Zuckerrübe, Baumwolle und Soja nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzungen.

Gegenstand der Erfindung sind daher auch herbizide Mittel, die gekennzeichnet sind durch einen Gehalt an einer Verbindung der Formel I in Kombination mit üblichen Formulierungshilfsmitteln und Inertstoffen sowie deren Verwendung im landwirtschaftlichen Bereich.

Die erfindungsgemäßen Mittel enthalten die Wirkstoffe der Formel I im allgemeinen zu 2 - 95 Gew.-%. Sie können als Spritzpulver, emulgierbare Konzentrate, versprühbare Lösungen, Stäubemittel oder Granulate in den üblichen Zubereitungen angewendet werden.

Die Spritzpulver sind in Wasser gleichmäßig dispergierbare Präparate, die neben dem Wirkstoff außer einem Verdünnungsmittel oder Inertstoff noch Netzmittel z.B. polyoxethylierte Alkylphenole, polyoxethylierte Fettalkohole, Alkyl- oder Alkylphenylsulfonate und Dispergiermittel, z.B. ligninsulfonsaures natrium, 2,2'-dinaphthylmethan-6,6'-disulfonsaures Natrium, dibutylnaphthalinsulfonsaures Natrium oder auch oleylmethyltaurinsaures Natrium enthalten.

Emulgierbare Konzentrate werden durch Auflösen des Wirkstoffes in einem organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt. Als Emulgatoren können beispielsweise verwandt werden: alkylarylsulfonsaure Calciumsalze wie Ca-dodecylbenzosulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Fettalkohol-Propylenoxid-Ethylenoxid-Kondensationsprodukte, Alkylpolyether, Sorbitanfettsäureester, Polyoxethylensorbitfettsäureester oder Polyoxethylensorbitester.

Stäubemittel erhält man durch Vermahlen des Wirkstoffes mit fein verteilten, festen Stoffen, z.B. Talkum, natürlichen Tonen, wie Kaolin, Bentonit, Pyrophillit oder Diatomeenerde.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln z.B. Polyvinylalkohol, polyacrylsaurem Natrium oder auch Mineralölen auf die Oberfläche von Trägerstoffen wie Sand, Kaolinite oder von granuliertem Inertmaterial. Auch können geeignete Wirkstoffe in der für die Herstellung von Düngemittelgranalien üblichen Weise - gewünschtenfalls in Mischung mit Düngemitteln - hergestellt werden.

Bei herbiziden Mitteln können die Konzentrationen der Wirkstoffe in den handelsüblichen Formulierungen verschieden sein.

In Spritzpulvern variiert die Wirkstoffkonzentration z.B. zwischen etwa 10 % und 80 %, der Rest besteht aus den oben angegebenen Formulierungszusätzen. Bei emulgierbaren Konzentraten kann die Wirkstoffkonzentration gleichfalls etwa 10 % bis 80 % betragen. Staubförmige Formulierungen enthalten etwa 2 - 20 %. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Zur Anwendung als Herbizide werden die handelsüblichen Konzentrate gegebenenfalls in üblicher Weise verdünnt, z.B. bei Spritzpulvern und emulgierbaren Konzentraten mittels Wasser. Staubförmige und granulierte Zubereitungen sowie versprühbare Lösungen werden vor der Anwendung nicht mehr mit weiteren inerten Stoffen verdünnt. Mit den äußeren Bedingungen wie Temperatur, Feuchtigkeit, u.a. variiert die erforderliche

Aufwandmenge. Sie beträgt im allgemeinen zwischen 0.01 und 10 kg/ha, vorzugsweise etwa 0.05 bis 2.0 kg/ha Wirkstoff.

Für einige Anwendungsgebiete kann es zweckmäßig sein, die neuen Herbizide mit einem oder mehreren Herbiziden gemeinsam anzuwenden, z.B. als Tankmischung oder in Form einer Fertigformulierung, um weitere vorteilhafte Wirkungen zu erzielen.

Die erfindungsgemäßen Wirkstoffe können mit anderen Herbiziden, Insektiziden und Fungiziden kombiniert werden.

## Formulierungsbeispiele

### Beispiel 1

Ein Stäubemittel wird erhalten, indem man a) 10 GT (GT = Gewichtsteile) Wirkstoff(e) und 90 GT Talkum oder einem anderen Inertstoff mischt und in einer Schlagmühle zerkleinert, oder indem man b) 60 GT Wirkstoff, 35 GT Talkum und 5 GT Haftmittel, z.B. ein Polysaccharid, in der gleichen Weise homogenisiert.

### Beispiel 2

Ein in Wasser leicht dispergierbares, benetzbares Pulver wird erhalten, indem man 25 GT Wirkstoff(e), 64 GT kaolinhaltigen Quarz als Inertstoff, 10 GT ligninsulfonsaures Kalium und 1 GT oleoylmethyltaurinsaures Natrium als Netz- und Dispergierhilfsmittel mischt und in einer Stiftmühle mahlt. Eine Formulierung mit 5 % Wirkstoffgehalt kann wie folgt zusammengesetzt sein: 5 % Wirkstoff(e), 6 % eines sulfonierten Napthalinformaldehydkondensat (z.B. (R)Dispersogen A der HOECHST AG), 2 % eines Na-Salzes einer Alkylnapthalinsulfonsäure (z.B. (R)Leonil DB der HOECHST AG), 5 % eines Gemisches aus Polypropylenglykol und $SiO_2$ (z.B. (R)Acrotin 341 der HOECHST AG), 25 % $SiO_2$ und 57 % Kaolin.

Beispiel 3

Ein in Wasser leicht dispergierbares Dispersionskonzentrat wird erhalten, indem man 20 GT Wirkstoff(e) mit 6 GT eines Alkylphenolpolyglykolethers, 3 GT Isotridecanolpolyglykolether (8 Ethylenoxid-Einheiten) und 71 GT paraffinischem Mineralöl (Siedebereich ca. 255 bis über 377°C) mischt und in einer Reibkugelmühle auf eine Feinheit von unter 5 um vermahlt.

Beispiel 4

Ein emulgierbares Konzentrat wird erhalten aus 15 GT Wirkstoff(e), 75 GT Cyclohexanon als Lösemittel und 10 GT oxethyliertem Nonylphenol (10 Ethylenoxid-Einheiten) als Emulgator.

Herstellungsbeispiele

Beispiel 1

2,2,2-Trichlor-1-ethoxy-ethanol

14,7 g (0,1 mol) Chloral wurden in 50 ml n-Hexan gelöst. Man tropfte bei 10-15°C 4,6 g (0,1 mol) Ethanol zu, ließ 4 Stunden bei Raumtemperatur rühren und kühlte im Eisbad, wobei weiterhin kräftig gerührt wurde. Die abgeschiedenen Kristalle wurden abgesaugt und mit wenig kaltem n-Hexan gewaschen. Man erhielt 16,8 g (87 % d.Th.) an 2,2,2-Trichlor-1-ethoxy-ethanol vom Fp. 46-47°C.

Beispiel 2

4-(2,2,2-Trichlor-1-hydroxy-ethoxy)-methyl-2,2-dimethyl-1,3-dioxolan

13,2 g (0,1 mol) 4-Hydroxymethyl-2,2-dimethyl-1,3-dioxolan

wurden zu einer Lösung von 14,7 g (0,1 mol) Chloral in 50 ml Essigester getropft, wobei die Temperatur unter 20°C gehalten wurde. Man ließ 24 Stunden bei Raumtemperatur rühren und destillierte das Lösungsmittel im Hochvakuum bei Raumtemperatur ab. Es blieb 4-(2,2,2-Trichlor-1-hydroxy-ethoxy)-methyl-2,2-dimethyl-1,3-dioxolan als Wachs zurück. Die Ausbeute betrug 27,4 g (98 % d.Th.). Das Produkt zersetzte sich langsam bei Raumtemperatur.

Beispiel 3

5-(2-Chlor-4-trifluormethylphenoxy)-2-nitro-benzoesäure

Zu einer Mischung aus 120 ml konz. Schwefelsäure und 200 ml 1,2-Dichlorethan wurde bei 0°C unter kräftigem Rühren 30,0 g (0,095 mol) 3-(2-Chlor-4-trifluormethylphenoxy)-benzoesäure zugegeben. Man kühlte auf -10°C und versetzte portionsweise mit 9,5 g (0,095 mol) Kaliumnitrat und 0,5 g Thallium (III)-trifluoracetat. Nach 0,5 Stunden bei 0°C ließ man das Reaktionsgemisch auf Raumtemperatur erwärmen, goß es auf 400 ml Eiswasser, extrahierte mit Chloroform, trocknete den Extrakt über $Na_2SO_4$ und engte im Vakuum ein. Man erhielt 27,8 g (81 % d.Th.) 5-(2-Chlor-4-trifluormethylphenoxy)-2-nitrobenzoesäure vom Fp. 150-155°C.

Beispiel 4

5-(2-Chlor-4-trifluormethylphenoxy)-2-nitrobenzoylchlorid

26 g (0,072 mol) 5-(2-Chlor-4-trifluormethylphenoxy)-2-nitrobenzoesäure wurden in 30 ml Thionylchlorid bei 80°C erhitzt, bis die Gasentwicklung beendet war (ca. 2 Stunden). Das überschüssige Thionylchlorid wurde im Vakuum abdestilliert und der Rückstand in n-Hexan ausgerührt. Nach Filtration erhielt man 26,2 g (95,8 % d.Th.) 5-(2-Chlor-4-trifluormethylphenoxy)-2-nitrobenzoylchlorid vom Fp. 64-65°C.

Beispiel 5

5-(2-Chlor-4-trifluormethylphenoxy)-2-nitrobenzoesäure-2,2,2-trichlor-1-ethoxyethylester

8 g (0,021 mol) 5-(2-Chlor-4-trifluormethylphenoxy)-2-nitrobenzoylchlorid wurden in 20 ml Tetrahydrofuran gelöst. Man tropfte bei -5°C zunächst 2,2,2-Trichlor-1-ethoxyethanol (4,1 g, 0,021 mol), in 20 ml Tetrahydrofuran gelöst, und darauf 3,0 ml Triethylamin zu. Man ließ 2 Stunden bei 5-10°C rühren und goß auf 100 ml Wasser. Nach Extraktion mit Diethylether und Einengen des getrockneten Extrakts wurden 6,6 g (58 % d.Th.) 5-(2-Chlor-4-trifluormethylphenoxy)-2-nitrobenzoesäure-2,2,2-trichlor-1-ethoxyethylester vom Fp. 102-104°C erhalten.

Beispiel 6

5-(2-Chlor-4-trifluormethylphenoxy)-2-nitrobenzoesäure-1-(2,2-dimethyl-1,3-dioxolan-4-yl)-methoxy-2,2,2-trichlorethylester

7,6 g (0,02 mol) 5-(2-Chlor-4-trifluormethylphenoxy)-2-nitrobenzoylchlorid wurden in 20 ml Tetrahydrofuran vorgelegt. Man tropfte bei -10°C 2,9 ml Triethylamin zu. Bei -5°C wurden 5,6 g (0,02 mol) 4-(2,2,2-Trichlor-1-hydroxy-ethoxy)-methyl-2,2-dimethyl-1,3-dioxolan, gelöst in 20 ml Tetrahydrofuran, zugegeben. Man ließ 1,5 Stunden bei 5-10°C rühren, saugte das entstandene Triethylammoniumchlorid ab, goß das Filtrat auf 100 ml Eiswasser und extrahierte mit Diethylether. Trocknung des Ethers über Natriumsulfat, Einengen und Ausrühren mit n-Hexan ergab 9,0 g (72 % d.Th.) 5-(2-Chlor-4-trifluormethylphenoxy)-2-nitrobenzoesäure-1-(2,2-dimethyl-1,3-dioxolan-4-yl)-methoxy-2,2,2-trichlorethylester vom Fp. 82-84°C.

Auf analoge Weise lassen sich die folgenden Verbindungen der Tabelle 1 herstellen

Tabelle 1:

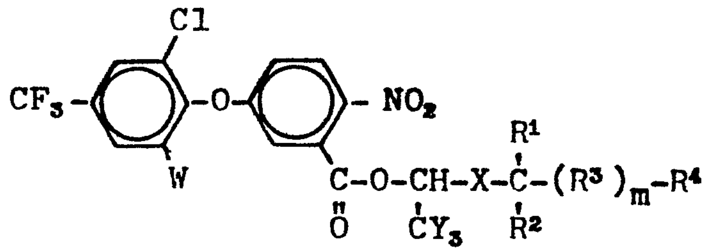

(I)

| Verb. Nr. | W | X | Y | | $R^1$ | $R^2$ | $R^3$ | $R^4$ | m | Fp. |
|---|---|---|---|---|---|---|---|---|---|---|
| 7 | H | O | Cl | | H | H | – | $CF_2CHF_2$ | 0 | Wachs |
| 8 | H | O | Cl | | H | H | $CH_2-O-CH_2$ | $CH_2CN$ | 1 | |
| 9 | H | O | Cl | | H | H | $CH_2-S-CH_2$ | $CH_2CN$ | 1 | Wachs |
| 10 | H | S | Cl | | H | H | $CH_2-S-CH_2$ | $CH_2CN$ | 1 | |
| 11 | H | O | Cl | | H | H | $CH_2-O-CH_2$ | $CH_2-NO_2$ | 2 | |
| 12 | H | O | Cl | | H | $CH_3$ | $CH_2$ | $CH_2-O-CH_3$ | 1 | |
| 13 | H | O | Cl | | H | H | $CH_2-O-CH_2$ | $CH_2-O-CH_3$ | 1 | |
| 14 | Cl | O | Cl | | $CF_3$ | $CF_3$ | – | H | 0 | |
| 15 | Cl | O | Cl | | $i-C_3H_7$ | H | – | $CH_2-O-CH_3$ | 0 | |
| 16 | H | O | Cl | | $CH_3$ | $CH_3$ | – | $CH_3$ | 0 | |
| 17 | H | O | F | | H | H | $CH_2OCH_2$ | $CH_2CN$ | 1 | |
| 18 | F | O | F | | H | $CH_3$ | – | $CF_2CHF_2$ | 0 | |
| 19 | F | S | Cl | | $CH_3$ | $CH_3$ | $CH_2$ | $CH_2OCH_3$ | 1 | |
| 20 | H | S | Cl | | H | H | $CH_2-S-CH_2$ | $CH_2CN$ | 1 | |
| 21 | F | S | Cl | | H | H | $CH_2-S-CH_2$ | $CH_2CN$ | 1 | |
| 22 | H | O | Cl | | H | H | – | $COOC_2H_5$ | 0 | Wachs |
| 23 | H | O | Cl | | H | $CH_3$ | – | $COOC_2H_5$ | 0 | Wachs |

./.

Fortsetzung Tabelle 1

| Verb. Nr. | W | X | Y | | $R^1$ | $R^2$ | $R^3$ | $R^4$ | m | Fp. |
|---|---|---|---|---|---|---|---|---|---|---|
| 24 | H | S | Cl | | H | H | - | $COOC_2H_5$ | 0 | Wachs |
| 25 | H | S | Cl | | H | $CH_3$ | - | $COOC_2H_5$ | 0 | Wachs |
| 26 | H | O | Cl | | H | $CH_3$ | - | $COOCH_2-C_6H_5$ | 0 | |
| 27 | H | O | Cl | | H | $CH_3$ | - | $COO(CH_2)_2OCH_3$ | 0 | |
| 28 | H | O | Cl | | H | H | $CH_2$ | $COOCH_3$ | 1 | |
| 29 | Cl | O | Cl | | H | $CH_3$ | $CH_2$ | $COOCH_3$ | 2 | |
| 30 | H | O | Cl | | $CH_3$ | $CH_3$ | - | $COOC_2H_5$ | 0 | Wachs |
| 31 | H | O | Cl | | $CH_2COOCH_3$ | H | - | $COOCH_3$ | 0 | Wachs |
| 32 | H | O | Cl | | $C_6H_5$ | H | - | $COOC_2H_5$ | 0 | Wachs |
| 33 | Cl | O | Cl | | H | H | - | $COOC_2H_5$ | 0 | Wachs |
| 34 | Cl | O | Cl | | $CH_3$ | H | - | $COOC_2H_5$ | 0 | Wachs |
| 35 | H | O | Cl | | $CH_2CN$ | $CH_3$ | $CH_2$ | $COOC_4H_9$ | 1 | |
| 36 | Cl | O | Cl | | H | $CH_3$ | - | $COOCH_2Cl$ | 0 | |
| 37 | H | O | Cl | | H | H | - | 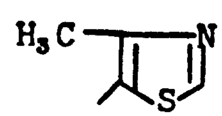 | 0 | Wachs |
| 38 | H | O | Cl | | H | H | - | 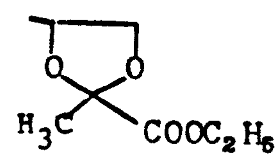 | 0 | |
| 39 | H | O | Cl | | H | 2-Cl-$C_6H_4$ | - | H | 0 | |

./.

Fortsetzung Tabelle 1

| Verb.-Nr. | W | X | Y | | $R^1$ | $R^2$ | $R^3$ | $R^4$ | m | Fp. |
|---|---|---|---|---|---|---|---|---|---|---|
| 40 | H | O | Cl | | $CH_3$ | $2-F-C_6H_4$ | - | H | 0 | |
| 41 | Cl | O | Cl | | H | 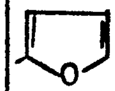 | - | $CH_3$ | 0 | |
| 42 | Cl | O | F | | H | $CH_3$ | - | COO-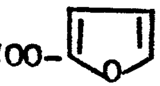 | 0 | |
| 43 | Cl | O | F | | H | H | - | $COO-CH_2-$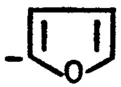 | 0 | |
| 44 | H | O | Cl | | H | H | - | $\overset{O}{\overset{\|}{P}}(OC_2H_5)_2$ | 0 | Wachs |
| 45 | H | O | Cl | | H | $CH_3$ | - | $\overset{O}{\overset{\|}{P}}(OCH_3)_2$ | 0 | |
| 46 | H | O | Cl | | H | H | - | $\overset{O}{\overset{\|}{P}}(CH_3)_2$ | 0 | 66-67 |
| 47 | H | O | Cl | | H | H | - | 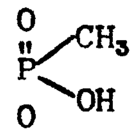 | 0 | |
| 48 | Cl | O | Cl | | $CH_3$ | $CH_3$ | - | $\overset{O}{\overset{\|}{P}}(OCH_3)_2$ | 0 | |
| 49 | Cl | O | F | | $CH_3$ | H | $CH_2$ | $COO-CH_2-\overset{O}{\overset{\|}{P}}(OCH_3)_2$ | 1 | |

./.

Fortsetzung Tabelle 1

| Verb.-Nr. | W | X | Y | | R¹ | R² | R³ | R⁴ | m | Fp. |
|---|---|---|---|---|---|---|---|---|---|---|
| 50 | H | O | Cl | | H | H | $CH_2$ | $CH_2\overset{O}{\overset{\|\|}{S}}CH_3$ | 1 | |
| 51 | Cl | O | Cl | | $CH_3$ | H | - | $CH_2SO_2CH_3$ | 0 | |
| 52 | H | O | Cl | | H | H | $CH_2$ | $CONH_2$ | 2 | |
| 53 | H | O | Cl | | H | $CH_3$ | - | $CON(CH_3)_2$ | 0 | |
| 54 | H | S | Cl | | H | $CH_3$ | - | $CONH(i)C_3H_7$ | 0 | |
| 55 | Cl | S | Cl | | $CH_3$ | $CH_3$ | $CH_2OCH_2$ | $CON(CH_3)_2$ | 1 | |
| 56 | Cl | O | Cl | | H | $CH_3$ | - | $CONHC_6H_5$ | 0 | |
| 57 | H | O | Cl | | H | H | - | $CONHCH_2C_6H_5$ | 0 | |
| 58 | H | O | Cl | | H | $CH_3$ | - | $COOC_6H_5$ | 0 | |
| 59 | H | O | Cl | | H | $CH_3$ | - | 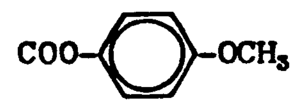 | 0 | |
| 60 | Cl | O | Cl | | H | $CH_3$ | - | 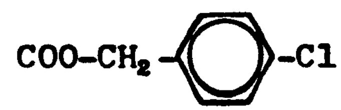 | 0 | |
| 61 | Cl | O | Cl | | H | $CH_3$ | - | $COO-(CH_2)_3-C_6H_5$ | 0 | |
| 62 | H | O | Cl | | H | $CH_2-COOCH_3$ | - | $COO-CH_2-C_6H_5$ | 0 | |
| 63 | H | O | F | | $CH_2Cl$ | 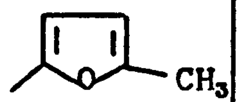 | - | $COOC_2H_5$ | 0 | |

./.

Fortsetzung Tabelle 1

| Verb.-Nr. | W | X | Y | | $R^1$ | $R^2$ | $R^3$ | $R^4$ | m | Fp. |
|---|---|---|---|---|---|---|---|---|---|---|
| 64 | H | O | Cl | | 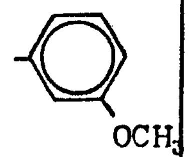 | $CF_3$ | - | $COOCH_3$ | 0 | |
| 65 | Cl | S | Cl | | $CH_2NO_2$ | H | $CH_2-S-CH_2$ | $COOCH_3$ | 2 | |
| 66 | F | O | Cl | | $CH_3$ | $CH_2OCH_3$ | - | $COOCH_3$ | 0 | |
| 67 | F | O | F | | $CF_3$ | H | H | $COOC_2H_5$ | 0 | |
| 68 | H | O | Cl | | H | $CH_3$ | - | $COOCH_2CF_2CH_2F$ | 0 | |
| 69 | H | O | Cl | | 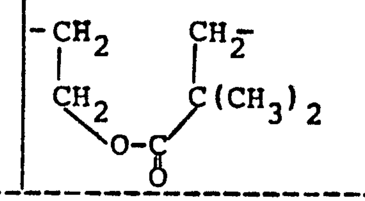 | | | | | |

Fortsetzung Tabelle 1

| Verb.-Nr. | W | X | Y | R¹ | R² | R³ | R⁴ | m | Fp. |
|---|---|---|---|---|---|---|---|---|---|
| 70 | H | O | Cl | 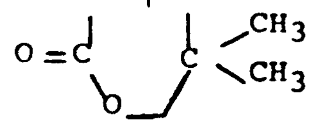 | | - | H | O | 65-67 |
| 71 | Cl | O | Cl | 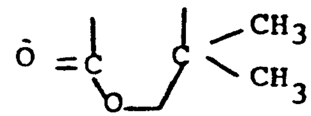 | | - | H | O | 95-97 |
| 72 | H | O | Cl | H | $CH_3$ | - | $\overset{\overset{O}{\|}}{P}(OC_2H_5)_2$ | O | Wachs |
| 73 | H | O | Cl | H | $COOCH_3$ | - | $\overset{\overset{O}{\|}}{P}(OC_2H_5)_2$ | O | Wachs |

## BIOLOGISCHE BEISPIELE

Die Schädigung der Unkrautpflanzen bzw. die Kulturpflanzenverträglichkeit wurde gemäß einem Schlüssel bonitiert, in dem die Wirksamkeit durch Wertzahlen von 0 - 5 ausgedrückt ist. Dabei bedeutet:

0 = ohne Wirkung bzw. Schaden
1 = 0 - 20 % Wirkung bzw. Schaden
2 = 20 - 40 % Wirkung bzw. Schaden
3 = 40 - 60 % Wirkung bzw. Schaden
4 = 60 - 80 % Wirkung bzw. Schaden
5 = 80 - 100 % Wirkung bzw. Schaden

### Beispiel 1

### Unkrautwirkung im Vorauflauf

Samen bzw. Rhizomstücke von mono- und dikotylen Unkrautpflanzen wurden in Plasstiktöpfen (Ø = 9 cm) in sandiger Lehmerde ausgelegt und mit Erde abgedeckt. Die in Form von benetzbaren Pulvern oder Emulsionskonzentraten formulierten erfindungsgemäßen Verbindungen wurden dann als wäßrige Suspensionen bzw. Emulsionen mit einer Wasseraufwandmenge von umgerechnet 600-800 l/ha in unterschiedlichen Dosierungen auf die Oberfläche der Abdeckerde appliziert. Nach der Behandlung wurden die Töpfe im Gewächshaus aufgestellt und unter guten Wachstumsbedingungen für die Unkrautpflanzen gehalten (Temperatur 23 plus/minus 1 Grad Celsius, relative Luftfeuchte 60-80 %).

Die optische Bonitur der Pflanzen- bzw. Auflaufschäden erfolgte nach dem Auflaufen der Versuchspflanzen nach einer Versuchszeit von 3-4 Wochen im Vergleich zu unbehandelten Kontrollen.

Wie die Boniturwerte in Tabelle 2 zeigen, weisen die erfindungsgemäßen Verbindungen eine gute herbizide Vorauflaufwirksamkeit gegen ein breites Spektrum von Ungräsern und Unkräutern auf.

Beispiel 2

Unkrautwirkung im Nachauflauf

Samen bzw. Rhizomstücke von mono- und dikotylen Unkräutern wurden in Plasstiktöpfen (Ø = 9 cm) in sandigem Lehmboden ausgelegt, mit Erde abgedeckt und im Gewächshaus unter guten Wachstumsbedingungen angezogen. Drei Wochen nach der Aussaat wurden die Versuchspflanzen im Dreiblattstadium behandelt. Die als Spritzpulver bzw. als Emulsionskonzentrate formulierten erfindungsgemäßen Verbindungen wurden in verschiedenen Dosierungen mit einer Wasseraufwandmenge von umgerechnet 600-800 l/ha auf die grünen Pflanzenteile gesprüht und nach ca. 3-4 Wochen Standzeit der Versuchspflanzen im Gewächshaus unter optimalen Wachstumsbedingungen (Temperatur 23°C +/- 1°C, relative Luftfeuchte 60-80 %) die Wirkung der Präparate optisch im Vergleich zu unbehandelten Kontrollen bonitiert.

Die erfindungsgemäßen Mittel weisen auch im Nachauflauf eine gute herbizide Wirksamkeit gegen ein breites Speltrum wirtschaftlich wichtiger Ungräser und Unkräuter auf (Tabelle 3).

Beispiel 3

Kulturpflanzenverträglichkeit

In weiteren Versuchen im Gewächshaus wurden Samen einer größeren Anzahl von Kulturpflanzen in Töpfen in sandigem Lehmboden ausgelegt und mit Erde abgedeckt.
Ein Teil der Töpfe wurde sofort, wie unter Bsp. 1 beschrieben, behandelt; die übrigen wurden im Gewächshaus aufgestellt, bis

die Pflanzen zwei bis drei echte Blätter entwickelt hatten und dann mit den erfindungsgemäßen Substanzen in unterschiedlichen Dosierungen, wie unter Bsp. 2 beschrieben, be sprüht.

Vier bis fünf Wochen nach der Applikation und Standzeit im Gewächshaus wurde mittels optischer Bonitur festgestellt, daß die erfindungsgemäßen Verbindungen zweikeimblättrige Kulturen wie z.B. Soja, Baumwolle, Raps, Zuckerrüben und Kartoffeln im Vor- und Nachauflaufverfahren selbst bei hohen Wirkstoffdosierungen ungeschädigt ließen. Einige Substanzen schonten darüber hinaus auch Gramineen-Kulturen wie z.B. Gerste, Sorghum, Mais, Weizen oder Reis. Die Verbindungen der Formel I weisen somit hohe Selektivität bei Anwendung zur Bekämpfung von unerwünschten Pflanzenwuchs in landwirtschaftlich wichtigen Kulturen auf.

TABELLE 2

Herbizide Wirkung der erfindungsgemäßen Verbindungen im Vorauflauf

| Verb. Nr. | Dosis (kg a.i./ha) | SIA | CRS | STM | ECG | AS |
|---|---|---|---|---|---|---|
| 5 | 2,5 | 5 | 5 | 2 | 3 | 2 |
| 6 | 2,5 | 2 | 3 | 0 | 0 | 1 |
| 7 | 2,5 | 3 | 5 | 2 | 5 | 3 |
| 9 | 2,5 | 4 | 5 | 3 | 4 | 4 |
| 22 | 2,5 | 5 | 5 | 2 | 5 | 4 |
| 23 | 2,5 | 5 | 5 | 5 | 5 | 5 |
| 30 | 2,5 | 5 | 5 | 2 | 5 | 5 |
| 31 | 2,5 | 5 | 5 | 2 | 5 | 5 |
| 34 | 2,5 | 5 | 5 | 5 | 1 | 2 |
| 37 | 2,5 | 4 | 3 | 1 | 2 | 2 |
| 46 | 2,5 | 5 | 5 | 1 | 5 | 4 |

TABELLE 3

Herbizide Wirkung der erfindungsgemäßen Verbindungen im Nachauflauf

| Verb. Nr. | Dosis (kg a.i./ha) | SIA | CRS | STM | ECG | AS |
|---|---|---|---|---|---|---|
| 5 | 2,5 | 5 | 4 | 2 | 2 | 1 |
| 6 | 2,5 | 4 | 4 | 3 | 0 | 1 |
| 7 | 2,5 | 5 | 5 | 5 | 3 | 2 |
| 9 | 2,5 | 5 | 4 | 0 | 2 | 1 |
| 22 | 2,5 | 5 | 5 | 3 | 5 | 5 |
| 23 | 2,5 | 5 | 5 | 5 | 2 | 1 |
| 30 | 2,5 | 5 | 5 | 5 | 4 | 5 |
| 31 | 2,5 | 5 | 5 | 5 | 3 | 4 |
| 34 | 2,5 | 5 | 5 | 5 | 4 | 5 |
| 37 | 2,5 | 5 | 4 | 0 | 2 | 2 |
| 46 | 2,5 | 5 | 5 | 2 | 2 | 2 |

Abkürzungen Tabelle 1 und 2

SIA = Sinapis arvensis
CRS = Chrysanthemum segetum
STM = Stellaria media
ECG = Echinochloa crus-galli
AS = Avena sativa
a.i. = Aktivsubstanz

0217101

Patentansprüche

1. Verbindungen der Formel I,

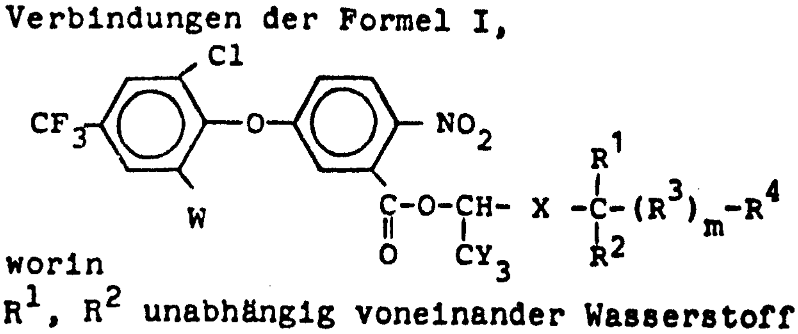

(I)

worin

$R^1$, $R^2$ unabhängig voneinander Wasserstoff $(C_1-C_4)$ Alkyl, das durch Halogen, Nitro, Cyano, $(C_1-C_4)$ Alkoxy, $(C_1-C_4)$Alkylthio, einen Rest der Formeln $-COR^6$, $-O-CO-R^6$ oder $-N(R^5)_2$ ein- oder mehrfach substituiert sein kann, Phenyl, Thienyl, Furanyl, die alle drei durch $(C_1-C_4)$Alkyl, Halogen, Nitro, Cyano, einen Rest der Formeln $-COOR^5$, $-OR^5$, $-S-R^5$, $-N(R^5)_2$ oder $-PO(XR^5)_2$ ein- oder mehrfach substituiert sein können, einen Rest der Formeln $-\overset{X}{\overset{\|}{C}}-R^6$ oder $-\overset{X}{\overset{\|}{C}}-XR^5$ oder

$R^1$, $R^2$ zusammen mit dem benachbarten C-Atom einen vier- bis siebengliedrigen gesättigten Kohlenwasserstoffring, bei dem eine oder zwei Methylengruppen durch $>C=O$ und/oder -O- ersetzt sein kann und welcher durch $(C_1-C_4)$ Alkyl ein- oder mehrfach substituiert sein kann,

$R^3$ einen Rest der Formeln $-(C(R^5)_2)_n-$, $-(CH_2)_n-X-(CH_2)_p-$ oder -X-

$R^4$ Wasserstoff, $(C_1-C_4)$ Alkyl, das durch Halogen, Cyano, Nitro, $(C_1-C_4)$ Alkylsulfinyl oder $(C_1-C_4)$ Alkylsulfonyl ein- oder mehrfach substituiert sein kann, einen Thiazolyl- oder Dioxolan-Rest, die beide durch $(C_1-C_4)$Alkyl und/oder $(C_1-C_4$ Alkoxy)-carbonyl substituiert sein können, einen Rest der Formeln $-X-R^5$,

$-\underset{X}{\underset{\|}{C}}-R^5$, $-\underset{X}{\underset{\|}{C}}-N(R^5)_2$, $-\underset{X}{\underset{\|}{C}}-NH-R^5$, $-\underset{X}{\underset{\|}{C}}-XR^5$,

$-\underset{\overset{\|}{X}}{P}(XR^5)_2$, $-\underset{\overset{\|}{X}}{P}(R^6)_2$, $-\underset{\overset{\|}{O}}{S}-R^6$, $-\underset{\overset{\|}{O}}{\overset{\underset{\|}{O}}{S}}-R^6$, $-CH_2-R^5$,

$R^5$ = Wasserstoff, ($C_1$-$C_4$) Alkyl, ($C_2$-$C_6$) Alkenyl, ($C_2$-$C_6$) Alkinyl, wobei die drei letztgenannten Reste durch Halogen, Nitro, Cyano, ($C_1$-$C_4$) Alkoxy, ($C_1$-$C_4$) Alkylthio, Di ($C_1$-$C_4$-alkyl)-amino, ($C_1$-$C_4$-Alkoxy)-carbonyl, Tri($C_1$-$C_4$-alkyl)silyl, Phenyl, Phenyl, das durch ($C_1$-$C_4$) Alkyl, Halogen, Nitro, Cyano, Carboxy, ($C_1$-$C_4$-Alkoxy)carbonyl, ($C_1$-$C_4$) Alkoxy oder Di-($C_1$-$C_4$-alkyl)amino substituiert ist, Furanyl, Thienyl oder Furanyl, Thienyl, die beide durch ($C_1$-$C_4$) Alkyl, Halogen oder ($C_1$-$C_4$) Alkoxy substituiert sind, substituiert sein können, Phenyl oder ein heterocyclischer 5-oder 6-Ring mit ein bis drei Heteroatomen aus der Gruppe Sauerstoff, Schwefel, Stickstoff als Ringglieder, wobei Phenyl oder der heterocyclische Ring durch ($C_1$-$C_4$) Alkyl, Halogen, Nitro, Cyano, ($C_1$-$C_4$)Alkoxy, ($C_1$-$C_4$) Alkylthio, ($C_1$-$C_4$-Alkoxy)-carbonyl, Carboxy, Amino, ($C_1$-$C_4$) Alkylamino, Di($C_1$-$C_4$-alkyl)amino oder $-PO(OC_1-C_4\text{-alkyl})_2$ substituiert sein kann, sowie für den Fall $R^4$= $-C(X)XR^5$ oder $P(X)\ (XR^5)_2$ ein in der Landwirtschaft einsetzbares Kation,

$R^6$ die für $R^5$ genannten Reste außer Wasserstoff

W Wasserstoff, Chlor oder Fluor

X O, S oder $NR^5$

Y Halogen oder Wasserstoff

m 0 oder 1

n 1,2 oder 3 und

p 0, 1, 2 oder 3 bedeuten.

2. Verfahren zur Herstellung der Verbindungen der Formel I,

dadurch gekennzeichnet, daß man eine Verbindung der Formel (II)

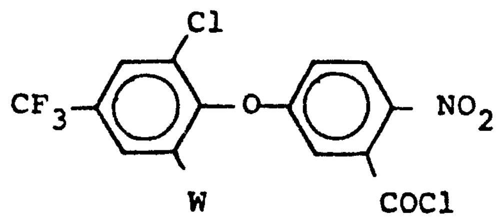

(II)

mit einer Verbindung der Formel (III)

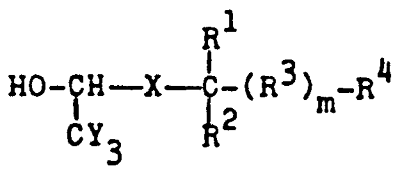

(III)

umsetzt und den entstehenden Chlorwasserstoff aus dem Reaktionsgemisch entfernt.

3. Herbizide Mittel, gekennzeichnet durch einen Gehalt an einer Verbindung der Formel I von Anspruch 1.

4. Verwendung von Verbindungen der Formel I von Anspruch 1 zur Bekämpfung von Schadpflanzen.

5. Verfahren zur Bekämpfung von Schadpflanzen, dadurch gekennzeichnet, daß man eine Verbindung der Formel I von Anspruch 1 auf diese oder die von ihnen bewachsenen Böden appliziert.

1. Verfahren zur Herstellung der Verbindungen der Formel I

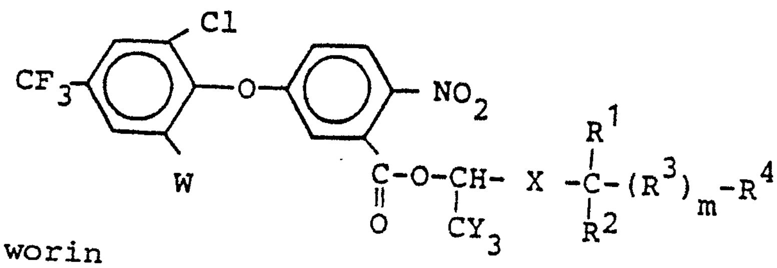

(I)

worin

$R^1$, $R^2$ unabhängig voneinander Wasserstoff $(C_1-C_4)$ Alkyl, das durch Halogen, Nitro, Cyano, $(C_1-C_4)$ Alkoxy, $(C_1-C_4)$Alkylthio, einen Rest der Formeln $-COR^6$, $-O-CO-R^6$ oder $-N(R^5)_2$ ein- oder mehrfach substituiert sein kann, Phenyl, Thienyl, Furanyl, die alle drei durch $(C_1-C_4)$Alkyl, Halogen, Nitro, Cyano, einen Rest der Formeln $-COOR^5$, $-OR^5$, $-S-R^5$, $-N(R^5)_2$ oder $-PO(XR^5)_2$ ein- oder mehrfach substituiert sein können, einen Rest der Formeln $-\overset{X}{\overset{\|}{C}}-R^6$ oder $-\overset{X}{\overset{\|}{C}}-XR^5$ oder

$R^1$, $R^2$ zusammen mit dem benachbarten C-Atom einen vier- bis siebengliedrigen gesättigten Kohlenwasserstoffring, bei dem eine oder zwei Methylengruppen durch $>C=O$ und/oder $-O-$ ersetzt sein kann und welcher durch $(C_1-C_4)$ Alkyl ein- oder mehrfach substituiert sein kann,

$R^3$ einen Rest der Formeln $-(C(R^5)_2)_n-$, $-(CH_2)_n-X-(CH_2)_p-$ oder $-X-$

$R^4$ Wasserstoff, $(C_1-C_4)$ Alkyl, das durch Halogen, Cyano, Nitro, $(C_1-C_4)$ Alkylsulfinyl oder $(C_1-C_4)$ Alkylsulfonyl ein- oder mehrfach substituiert sein kann, einen Thiazolyl- oder Dioxolan-Rest, die beide durch $(C_1-C_4)$Alkyl und/oder $(C_1-C_4$ Alkoxy)-carbonyl substituiert sein können, einen Rest der Formeln $-X-R^5$,

$-\underset{X}{\underset{\|}{C}}-R^5$, $-\underset{X}{\underset{\|}{C}}-N(R^5)_2$, $-\underset{X}{\underset{\|}{C}}-NH-R^5$, $-\underset{X}{\underset{\|}{C}}-XR^5$,

$-\underset{\underset{X}{\|}}{P}(XR^5)_2$, $-\underset{\underset{X}{\|}}{P}(R^6)_2$, $-\underset{\underset{O}{\|}}{S}-R^6$, $-\overset{\overset{O}{\|}}{\underset{\underset{O}{\|}}{S}}-R^6$, $-CH_2-R^5$,

$R^5$ = Wasserstoff, $(C_1-C_4)$ Alkyl, $(C_2-C_6)$ Alkenyl, $(C_2-C_6)$ Alkinyl, wobei die drei letztgenannten Reste durch Halogen, Nitro, Cyano, $(C_1-C_4)$ Alkoxy, $(C_1-C_4)$ Alkylthio, Di $(C_1-C_4$-alkyl)-amino, $(C_1-C_4$-Alkoxy)-carbonyl, Tri$(C_1-C_4$-alkyl)silyl, Phenyl, Phenyl, das durch $(C_1-C_4)$ Alkyl, Halogen, Nitro, Cyano, Carboxy, $(C_1-C_4$-Alkoxy)carbonyl, $(C_1-C_4)$ Alkoxy oder Di-$(C_1-C_4$-alkyl)amino substituiert ist, Furanyl, Thienyl oder Furanyl, Thienyl, die beide durch $(C_1-C_4)$ Alkyl, Halogen oder $(C_1-C_4)$ Alkoxy substituiert sind, substituiert sein können, Phenyl oder ein heterocyclischer 5 oder 6-Ring mit ein bis drei Heteroatomen aus der Gruppe Sauerstoff, Schwefel, Stickstoff als Ringglieder, wobei Phenyl oder der heterocyclische Ring durch $(C_1-C_4)$ Alkyl, Halogen, Nitro, Cyano, $(C_1-C_4)$Alkoxy, $(C_1-C_4)$ Alkylthio, $(C_1-C_4$-Alkoxy)-carbonyl, Carboxy, Amino, $(C_1-C_4)$ Alkylamino, Di$(C_1-C_4$-alkyl)amino oder $-PO(OC_1-C_4\text{-alkyl})_2$ substituiert sein kann, sowie für den Fall $R^4= -C(X)XR^5$ oder $P(X)(XR^5)_2$ ein in der Landwirtschaft einsetzbares Kation,

$R^6$ die für $R^5$ genannten Reste außer Wasserstoff

W Wasserstoff, Chlor oder Fluor

X O, S oder $NR^5$

Y Halogen oder Wasserstoff

m 0 oder 1

n 1,2 oder 3 und

p 0, 1, 2 oder 3 bedeuten,

dadurch gekennzeichnet, daß man eine Verbindung der Formel (II)

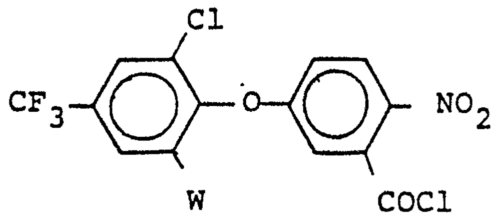

(II)

mit einer Verbindung der Formel (III)

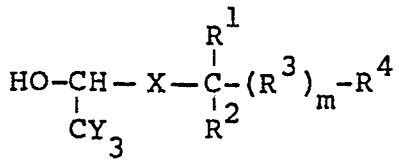

(III)

umsetzt und den entstehenden Chlorwasserstoff aus dem Reaktionsgemisch entfernt.

2. Herbizide Mittel, gekennzeichnet durch einen Gehalt an einer Verbindung der Formel I von Anspruch 1.

3. Verwendung von Verbindungen der Formel I von Anspruch 1 zur Bekämpfung von Schadpflanzen.

4. Verfahren zur Bekämpfung von Schadpflanzen, dadurch gekennzeichnet, daß man eine Verbindung der Formel I von Anspruch 1 auf diese oder die von Ihren bewachsenen Böden appliziert.

Europäisches Patentamt

# EUROPÄISCHER RECHERCHENBERICHT

Nummer der Anmeldung: 0217101

EP 86111563.2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| D,A | EP - A1 - 0 080 312 (ROHM AND HAAS COMPANY)<br>* Zusammenfassung; Beispiel 11*<br>-- | 1,3,4 | C 07 C 79/46<br>C 07 C 76/00<br>C 07 C 121/34<br>C 07 C 120/00<br>C 07 C 103/34<br>C 07 C 102/00<br>C 07 C 149/20<br>C 07 F 9/40<br>C 07 D 317/32<br>C 07 D 277/24<br>C 07 C 147/14<br>C 07 C 147/02<br>C 07 D 307/42 |
| A | DE - A1 - 2 950 401 (BAYER AG)<br>* Ansprüche *<br>-- | 1-5 | |
| A | DE - A1 - 3 210 056 (BAYER AG)<br>* Ansprüche *<br>-- | 1-5 | |
| A | EP - A1 - 0 028 277 (ROHM AND HAAS COMPANY)<br>* Zusammenfassung; Ansprüche; Beispiel 1 *<br>---- | 1-5 | |

RECHERCHIERTE SACHGEBIETE (Int. Cl 4)

C 07 C 79/00
C 07 C 121/00
C 07 C 103/00
C 07 C 149/00
C 07 F
C 07 D 317/00
C 07 D 277/00
C 07 C 147/00
C 07 D 307/00

Der vorliegende Recherchenbericht wurde für alle Patentansprüche erstellt.

| Recherchenort | Abschlußdatum der Recherche | Prüfer |
|---|---|---|
| WIEN | 28-11-1986 | KÖRBER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82

Europäisches Patentamt

# EUROPÄISCHER RECHERCHENBERICHT

0217101

EP 86111563.2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| | | | C 07 C 103/175<br>A 01 N 37/48 |
| | | | RECHERCHIERTE SACHGEBIETE (Int Cl 4) |

Der vorliegende Recherchenbericht wurde für alle Patentansprüche erstellt.

| Recherchenort | Abschlußdatum der Recherche | Prüfer |
|---|---|---|
| WIEN | 28-11-1986 | KÖRBER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1503 03 82